Europäisches Patentamt

European Patent Office

Office européen des brevets

(19)

(11) Veröffentlichungsnummer: **0 288 071 B1**

(12) **EUROPÄISCHE PATENTSCHRIFT**

(45) Veröffentlichungstag der Patentschrift: **08.07.92**

(51) Int. Cl.⁵: **C04B 38/10**, C04B 41/45, C22C 1/08, C12N 11/14

(21) Anmeldenummer: **88106442.2**

(22) Anmeldetag: **22.04.88**

Die Akte enthält technische Angaben, die nach dem Eingang der Anmeldung eingereicht wurden und die nicht in dieser Patentschrift enthalten sind.

(54) **Künstliche Steine und Verfahren zur Herstellung derselben.**

(30) Priorität: **24.04.87 DE 3713735**
**09.06.87 DE 3719153**
**22.08.87 DE 3743581**
**03.09.87 DE 3729371**
**24.04.87 DE 8705957 U**
**17.02.88 DE 3804884**

(43) Veröffentlichungstag der Anmeldung:
**26.10.88 Patentblatt 88/43**

(45) Bekanntmachung des Hinweises auf die Patenterteilung:
**08.07.92 Patentblatt 92/28**

(84) Benannte Vertragsstaaten:
**AT BE CH DE ES FR GB GR IT LI LU NL SE**

(56) Entgegenhaltungen:
**WO-A-85/04861          DE-A- 1 135 815**
**DE-A- 1 933 321        DE-A- 2 424 623**
**DE-A- 3 344 769        US-A- 3 497 455**
**US-A- 4 158 685**

**CHEMICAL ABSTRACTS, Band 102, 1985, Seite 286, Zusammenfassung Nr. 83387e, Columbus, Ohio, US; & JP-A-59 184 761 (INA SEITO CO., LTD) 20-10-1984**

(73) Patentinhaber: **Broggini, Arturo**
**Glockenstrasse 24**
**W-4000 Düsseldorf 30(DE)**

(72) Erfinder: **Broggini, Arturo**
**Glockenstrasse 24**
**W-4000 Düsseldorf 30(DE)**

(74) Vertreter: **Fitzner, Ulrich, Dr.**
**Am Eichförstchen 2a**
**W-4030 Ratingen 4(DE)**

**Beschreibung**

Gegenstand der vorliegenden Erfindung ist ein künstlicher Stein, der leicht und stabil ist, gegen Frost, Feuer und Witterung beständig ist und ein hohes Wärmedämmvermögen aufweist.

Steine, die teilweise die genannten Eigenschaften, zum Beispiel Schaumton, aufweisen, sind unter der Bezeichnung "Poroton" im Handel erhältlich. Es handelt sich hierbei um porige Hohllochziegel für Mauerwerk. Diese Ziegel sind wärmedämmend, schützen vor teuren Engergieverlusten und sorgen für ein ausgeglichenes Raumklima. Solche Ziegel entsprechen auch statischen Anforderungen. Nachteilig ist jedoch, daß diese Steine immer noch ein relativ hohes Gewicht haben. Außerdem ist die Herstellung mit Umweltbelastungen verbunden, weil Styropor oder andere Schaumkunststoffe zur Erzeugung der Poren gebrannt werden. Auch Blähton hat teilweise die genannten Eigenschaften. Er findet jedoch nur Anwendung als Granulat. Die Erstellung von Gußelementen ist nur durch Zusatz von Bindemitteln möglich.

Ferner sind mit Sägemehl und anderen Füllstoffen erstellte Leichtbausteine bekannt. Diese haben jedoch Nachteile, die darin zu sehen sind, daß Rückstände in Form von Asche nach dem Brand Salze bilden.

Aus der WO 85/04861 sind ferner Leichtkeramikmaterialien für Bauzwecke bekannt, welche durch Brennen einer geschäumten Mischung von Ton ggf. Zuschlagsstoffen, Tensiden, Verflüssigern und ggf. einem hydraulischen Binder hergestellt werden.

Schließlich ist aus der US-A-4 158 685 ein geschäumter Feuerfestkörper bekannt, welcher durch Vermischen eines aus Wasser, einem Flokkungshilfsmittel, feinverteiltem Feuerfestmaterial hergestellten Schlickers mit einem aus Wasser. Luft und Schaumbildnern hergestellten Schaum hergestellt werden. Aber auch diese Verfahren haben die bereits oben geschilderten Nachteile.

Aufgabe der Erfindung ist es demzufolge, einen durch Trocknen und ggf. Brennen hergestellten künstlichen Stein bestehend aus Schaumbaustoff und weiteren Zusatzstoffen zur Verfügung zu stellen, der die aufgezeigten Nachteile nicht hat und vielseitig verwendbar ist.

Diese Aufgabe wird dadurch gelöst, daß der Schaumbaustoff ein Gemisch aus a) durch Mischen von Seife, Wasser, Tensiden und Bariumcarbonat erzeugtem Schaum und b) Ton, Gips, Zement, Kalk, Metall und/oder Metallegierungen ist, und die Zusatzstoffe Bentonit, Titandioxid und Bariumcarbonat sind.

Als Schaumbaustoffe für gebrannte Steine werden vor allem Schaumton und Schaumkeramik bevorzugt. Gemische aus Schaumkeramik, Schaumton, Schaumgips, Schaumzement, Schaumkalk und Schaummetall weisen ein geringeres Gewicht im Vergleich zu den gelochten Ausführungen nach dem Stand der Technik auf. Als Zusatzstoffe können Titandioxid, Bariumcarbonat, Bentonit, tonhaltiges Material und Mineralfasern eingesetzt werden. Durch Imprägnierungsmittel, Kunststoffe und Reaktionsharze kann eine Erhöhung der Festigkeit erreicht werden.

Die erfindungsgemäßen Steine weisen ein geringes Gewicht auf. Sie sind daher für die Erstellung von Bauelementen der verschiedensten Art geeignet. Sie sind insbesondere auch für Möbelteile verwendbar. Das Material ist beständig gegen Hitze und Feuer, gegen Kälte, UV-Strahlen und sonstige Witterungseinflüsse. Ebenso zeichnet sich das erfindungsgemäße gebrannte Material besonders durch seine Beständigkeit gegen Frost und verschiedene Chemikalien aus.

Die erfindungsgemäßen Steine eignen sich auch als Leichtbausteine. Diese weisen nicht die Nachteile der bisher bekannten Leichtbausteine auf und ermöglichen eine Anwendung in vielen Bereichen. Sie können für belastetes und unbelastetes Mauerwerk verwendet werden und bieten eine entsprechend hohe Wärmedämmung. Die ausbrennbaren Bestandteile sind sehr viel geringer als bisher bekannt und hinterlassen keine Rückstände. Die Emulsionen sind daher schwer entflammbar, raumsparend, umweltfreundlich und feuchtigkeitsunempfindlich. Die aufgeschäumte Emulsion bildet eine glatte Schalenstruktur im Gemenge, die durch Zusatz verschiedener Stoffe beliebig gehärtet oder auch poröser gemacht werden kann und wird beim Ofenbrand ausgebrannt. Das Material ist leicht zu sägen und zu transportieren. Die Schaumbaustoffe unterscheiden sich hier von herkömmlichen Leichtbaustoffen durch ihre offenporige Gestaltung, die nicht mit Füllstoffen, sondern mit Schaum erzeugt wird.

Aufgrund des geringen Gewichts und der Stabilität sowie der Feuer- und Frostbeständigkeit sind die erfindungsgemäßen Schaumbaustoffe auch besonders für die Erstellung von Filteranlagen in Innen- und Außenräumen geeignet. Die Tatsache, daß das Material UV-strahlenbeständig ist, ermöglicht seine Anwendbarkeit mit einer langen wartungsfreien Lebensdauer. Insbesondere für Anlagen mit geringen Belastungsmöglichkeiten bieten Schaumbaustoffe aufgrund des geringen Gewichts neue Gestaltungsmöglichkeiten. Die einzelnen Steine können hierbei mit verschiedenen handelsüblichen Bindemitteln zusammengefügt werden.

Gegenstand der vorliegenden Erfindung ist ferner ein Verfahren zur Herstellung eines künstlichen Steins.

Das Verfahren ist dadurch gekennzeichnet, daß ein Schaumstoff durch Mischen von Seifen, Wasser, Tensiden und Bariumcarbonat erzeugt wird, der Schaum anschließend mit lehmiger Tonmasse, bzw. Ton oder Gips, Zement, Kalk, Metalloxiden gemischt wird, das Gemisch in eine Form gegossen wird, getrocknet und anschließend bei Verwendung von lehmigem Ton, Ton, Kalk und Metalloxiden im Ofen gebrannt wird. Das Trocknen wird vorzugsweise im Heißluftstrom durchgeführt.

Durch verschiedene Zusatzstoffe kann die chemische Affinität zwischen den einzelnen Komponenten erhöht werden. Die Festigkeit kann durch Zusatzstoffe ebenfalls erhöht werden, insbesondere durch Tränkung in verschiedenen Imprägnierungsmitteln, wie Kunststoffen, Reaktionsharzen, flüssigem Glas und Mineralstoffen. Die Zugabe von Gips hat zur Folge, daß sich schon nach etwa einer halben Stunde das flüssige Gemisch versteift.

Die Verwendung der erfindungsgemäßen künstlichen Steine liegt vor allem im Bausektor. Beispielsweise sind die Steine als Bausteine, Bauplatten und Isolierfassadenplatten einsetzbar. Aus dem erfindungsgemäßen Steinmaterial können jedoch auch Möbel gefertigt werden. Ebenso ist das Material für Natursteinimitationen geeignet. Ein weiteres Anwendungsgebiet sind der Bau von Feuerschutzelementen, Brunnenanlagen, Öfen, Filteranlagen, Biotopen, Riffbau, Füllstoffen und Granulaten sowie die Erstellung von künstlichen Knochen und Gelenken, sowie die Kieferchirurgie.

Schließlich können die erfindungsgemäßen Steine als Trägermaterial oder Füllmaterial in Reaktoren, insbesondere Festbettreaktoren eingesetzt werden. Das Trägermaterial ist vor allem für die Immobilisierung von Biokatalysatoren geeignet. Beispiele für solche Biokatalysatoren sind Enzyme, pflanzliche oder tierische Zellen sowie Mikroorganismen. Immobilisierungen mit den erfindungsgemäßen Steinen kommen bei den verschiedensten Bioverfahren, insbesondere aber in der Abwasserreinigung in Betracht.

**Patentansprüche**

1. Künstlicher, durch Trocknen und ggf. Brennen hergestellter Stein, bestehend aus Schaumbaustoff und weiteren Zusatzstoffen,
**dadurch gekennzeichnet,** daß der Schaumbaustoff ein Gemisch aus
a) durch Mischen von Seife, Wasser, Tensiden und Bariumcarbonat erzeugtem Schaum und
b) Ton, Gips, Zement, Kalk, Metall und/oder Metallegierungen ist, und die Zusatzstoffe Bentonit, Titandioxid und Bariumcarbonat sind.

2. Stein gemäß Anspruch 1,
**dadurch gekennzeichnet,** daß der Schaumbaustoff Mineralfasern enthält.

3. Stein gemäß einem der Ansprüche 1 oder 2,
**dadurch gekennzeichnet,** daß er mit Kunststoffen, Reaktonsharzen und/oder Imprägnierungsmitteln getränkt oder beschichtet ist.

4. Verfahren zur Herstellung künstlicher Steine durch
a) vorheriges Erzeugen eines Schaums aus einem seifenhaltigen Gemisch,
b) Vermischen des erhaltenen Schaums mit Ton, Gips, Zement, Kalk oder Metalloxiden,
c) Ausformen des so erhaltenen Gemischs,
d) Trocknen und
e) erforderlichenfalls Brennen der getrockneten Masse,
**dadurch gekennzeichnet,** daß der Schaum
durch Mischen von Seife, Wasser, Tensiden und Bariumcarbonat erzeugt und unter Zugabe von Wasser mit Ton, Gips, Zement und/oder Kalk gemischt wird.

5. Verfahren nach Anspruch 4,
**dadurch gekennzeichnet,** daß dem Gemisch aus Schaum und Ton, Gips, Zement und/oder Kalk als Zusatzstoffe Bentonit, Titandioxid und Bariumcarbonat zugesetzt werden.

6. Verfahren nach Anspruch 4,
**dadurch gekennzeichnet,** daß Mineralfasern zugesetzt werden.

7. Verfahren nach einem der Ansprüche 4 bis 6,
**dadurch gekennzeichnet,** daß zur Erhöhung der Festigkeit das gebrannte oder gebunde Material in flüssige Harze, Imprägnierungsmittel oder Glas getaucht wird.

**Claims**

1. Artificial stone consisting of a foamed building material and further additives and manufactured by drying and optionally calcining, characterized in that the foamed building material is a mixture of
(a) a roam generated by mixing soap, water, surface-active agents and barium carbonate and
(b) clay, gypsum, cement, lime, metal and/or metal alloys, and the additives are bentonite, titanium dioxide and barium carbonate.

2. A stone according to claim 1, characterized in that the foamed building material contains mineral fibres.

3. A stone according to any of the claims 1 or 2, characterized in that it is soaked or coated with plastic materials reaction resins and/or impregnating agents.

4. A process for the manufacture of artificial stones by
    (a) primarily generating a foam from a mixture containing soap;
    (b) mixing the resulting foam with clay, gypsum, cement, lime or metal oxides,
    (c) moulding the mixture obtained thereby,
    (d) drying, and
    (e) if necessary calcining of the dryed mass, characterized in that a foam is generated by mixing soap, water, surface-active agents and barium carbonate and is mixed, with the addition of water, with clay, gypsum, cement and/or lime.

5. A process according to claim 4, characterized in that bentonite, titanium dioxide and barium carbonate are added as additives to the mixture of foam and clay, gypsum, cement and/or lime.

6. A process according to claim 4, characterized in that mineral fibres are added.

7. A process according to any of the claims 4 to 6, characterized in that the calcined or bound material is immersed into liquid resins, impregnating agents or glass in order to increase the strength.

**Revendications**

1. Pierre artificielle fabriquée par séchage et éventuellement cuisson, composée d'un matériau de construction en mousse et d'autres additifs, caractérisée en ce que le matériau de construction en mousse est un mélange des substances suivantes :
    a) une mousse produite en mélangeant du savon, de l'eau, des tensio-actifs et du carbonate de baryum, et
    b) de l'argile, du plâtre, du ciment, de la chaux, un métal et/ou des alliages métalliques, et les additifs sont de la bentonite, du dioxyde de titane et du carbonate de baryum.

2. Pierre selon la revendication 1, caractérisée en ce que le matériau de construction en mousse contient des fibres minérales.

3. Pierre selon une des revendications 1 et 2, caractérisée en ce qu'elle est imprégnée ou revêtue de matières plastiques, de résines réactives et/ou de fluides d'imprégnation.

4. Procédé de fabrication de pierres artificielles consistant à :
    a) produire préalablement une mousse à partir d'un mélange contenant du savon,
    b) mélanger la mousse obtenue avec de l'argile, du plâtre, du ciment, de la chaux ou des oxydes métalliques,
    c) démouler le mélange ainsi obtenu,
    d) sécher et
    e) éventuellement cuire la masse séchée, caractérisé en ce que la mousse est produite en mélangeant du savon, de l'eau, des tensio-actifs et du carbonate de baryum et mélangée avec addition d'eau, avec de l'argile, du plâtre, du ciment et/ou de la chaux.

5. Procédé selon la revendication 4, caractérisé en ce qu'on ajoute de la bentonite, du dioxyde de titane et du carbonate de baryum comme additifs au mélange de mousse et d'argile, plâtre, ciment et/ou chaux.

6. Procédé selon la revendication 4, caractérisé en ce qu'on ajoute des fibres minérales.

7. Procédé selon une des revendications 4 à 6, caractérisé en ce que, pour améliorer la solidité, on plonge la matière cuite ou liée dans des résines liquides, des fluides d'imprégnation liquide ou du verre.